# EUROPÄISCHE PATENTSCHRIFT

(11) **EP 0 374 497 B1**
(45) Veröffentlichungstag und Bekanntmachung des Hinweises auf die Patenterteilung: **03.02.1993**
(21) Anmeldenummer: 89121383.7
(22) Anmeldetag: 18.11.1989
(51) Int. Cl.: C07C 323/12, C08G 65/32, C08G 65/20

(54) **Polybutandiol(1,4)w,w'bismercaptane und ihre Herstellung**
Polybutanediol(1,4)w,w'bismercaptans and their preparation
Polybutanediol(1,4)w,w'bismercaptanes et leur préparation

(30) Priorität: 29.11.1988 DE 3840204
(43) Veröffentlichungstag der Anmeldung: 27.06.1990
(73) Patentinhaber: BASF Aktiengesellschaft, 67063 Ludwigshafen (DE)
(72) Erfinder: Koehler, Ulrich, Dr., D-6900 Heidelberg (DE); Siegel, Hardo, Dr., D-6720 Speyer (DE); Hickmann, Eckhard, Dr., D-6701 Dannstadt-Schauernheim (DE)

(56) Entgegenhaltungen:
- GB-A- 962 862
- GB-A- 1 077 997
- US-A- 3 824 197
- US-A- 4 259 474

## Beschreibung

Diese Erfindung betrifft neue Polybutandiol(1,4)ω,ω′bismercaptane und ein Verfahren zu ihrer Herstellung.

Polytetrahydrofuran und Derivate des Polytetrahydrofurans haben als Zwischenprodukte für die Herstellung von Polymeren großes Interesse gefunden. Besonders gesucht sind hierbei Polytetrahydrofuranderivate mit endständigen reaktiven Gruppen wie Polytetrahydrofuran-ω,ω′-diamine (US-PS 3 824 198), die den Einbau der Polybutandiol-Kette in Polymere ermöglichen.

Gegenstand dieser Erfindung sind neue Polybutandiolderivate, die sich wegen ihrer vorteilhaften Eigenschaften, z.B. als Regler oder Vernetzer bei Polymerisationen für die Herstellung von Kunststoffen eignen. Diese neuen Verbindungen haben die allgemeine Formel

HS-(CH₂)₄-O-[(CH₂)₄-O]ₙ-(CH₂)₄-SH I,

in der n eine Zahl von 1 bis 150, vorzugsweise 2 bis 70, insbesondere 2 bis 55, bedeutet.

In Beispiel 3 der US-PS 3 824 197 werden ähnliche Schwefelatome enthaltende Polyether beschrieben, die durch Polymerisation von Tetrahydrofuran in Gegenwart von Trifluormethansulfonsäureanhydrid und Umsetzung der dabei erhaltenen Polytetrahydrofuranderivate mit Schwefelwasserstoff in Pyridin erhalten werden. Diese schwefelhaltigen Polytetrahydrofuranderivate haben jedoch Molmassen in der Größenordnung von 100 000. Außerdem ist ihr Schwefelgehalt erheblich größer als der Schwefelgehalt, den die entsprechenden Polybutandiole mit endständigen Thiolgruppen aufweisen würden.

Die neuen Butandiolderivate der Formel I werden z.B. dadurch hergestellt, daß man Polytetrahydrofuranderivate der allgemeinen Formel 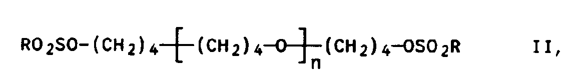
in der R einen Alkylrest mit 1 bis 4 C-Atomen oder einen Arylrest bedeutet und n die obengenannte Bedeutung hat, mit Schwefel abgebenden Verbindungen bei Temperaturen bis 150°C, vorzugsweise 50 bis 100°C, umsetzt.

In den Polytetrahydrofuranderivaten der Formel II steht R für einen Alkylrest, der 1 bis 4 C-Atome aufweist, oder für einen Arylrest, wie den Benzol- oder Toluolrest. Verbindungen der Formel II sind somit z.B. das in der JP-PS 70/16443 beschriebene Polytetrahydrofuran-bis-p-toluolsulfonat oder die in der älteren deutschen Patentanmeldung P 38 34 265.0 beschriebenen Polytetrahydrofuran-bisalkylsufonate.

Man setzt die Polytetrahydrofuranderivate der Formel I mit den Schwefel abgebenden Stoffen bei Temperaturen bis 150°C, vorzugsweise 50 bis 100°C, um. Schwefel abgebende Stoffe sind z.B. Schwefelwasserstoff, Sulfide, Hydrogensulfide, Thiosulfate, Thiocyanate, Xantogenate, Dithiocarbamate oder Thioharnstoff. Die Schwefel abgebenden Stoffe werden zweckmäßigerweise in stöchiometrischen Mengen eingesetzt, wobei man jedoch auch überschüssige Mengen, z.B. die bis zu doppelte Menge, anwenden kann. Die Umsetzung nimmt man vorzugsweise in einem Lösungsmittel vor, das unter den Umsetzungsbedingungen nicht in unerwünschter Weise mit den Reaktionsteilnehmern reagiert. Geeignete Lösungsmittel sind z.B. Alkohole, wie Ethanol, Methanol und Butanol oder Wasser, Tetrahydrofuran, Dioxan, Dimethylformamid, Dimethylsulfoxid und N-Methylpyrrolidon.

Besonders gute Ergebnisse werden erzielt, wenn man das Polytetrahydrofuranderivat der Formel II mit Thioharnstoff umsetzt. Man erhält hierbei die Polybutandiol(1,4)ω,ω′bismercaptane mit besonders hohen Selektivitäten.

### Beispiel 1

In einer mit Rückflußkühler und Tropftrichter versehenen 250 ml Rührapparatur werden unter Stickstoff 39 g (0,05 mol) Polytetrahydrofuran-bis-methansulfonat (siehe Formel II mit n = 6,7 und R = CH₃) in 50 ml Ethanol (95 proz.) gelöst. Man gibt 7,7 g Thioharnstoff (0,1 mol) zu und erhitzt das Gemisch 3 h zum Sieden. Dabei wird die anfänglich trübe Suspension klar. Nach Zutropfen von 6 g (0,15 mol) NaOH in 60 ml Wasser hält man das Gemisch weitere 2 h am Sieden und läßt es dann auf Raumtemperatur abkühlen. Man säuert mit 7,5 ml 30 proz. Schwefelsäure an (pH 1) und trennt die obere, organische Phase ab. Sie wird mit Methyl-tert.-butylether (MTB) verdünnt und über Na₂SO₄ getrocknet. Nach Einengen (60°C/50 mbar) erhält man 28,7 g (87,5 %) Polybutandiol (1,4)ω,ω′bismercaptan der Formel I mit n = 6,7 als gelbes Öl in > 90%iger Reinheit.

### Analysen:

Molekulargewicht (osm.): 700
¹H-NMR (CDCl₃): δ = 3,4 (m, Polyether, CH₂) 2,56 (m,α-CH₂), 1,6 (m, Polyether-CH₂), 1,35 (t, SH)
¹³C-NMR (CDCl₃): δ = 70,6 (Polyether-C), 70,1 (δ-C zur SH-Gruppe), 30,9 (β-C), 28,6 (γ-C) , 26,7 (Polyether-C), 244 (α-C zur SH-Gruppe)
IR(Film): 2939, 2856, 2796, 2565, 1483, 1447, 1436, 1368, 1208, 1114, 1017 cm¹.

Gemäß obigen Daten beträgt der Gehalt des bei der Reaktion entstandenen dimeren Nebenproduktes der Formel

HS-(CH₂)₄-O-[(CH₂)₄-O]ₙ-(CH₂)₄-S-(CH₂)₄-O-[(CH₂)₄-O]ₙ-(CH₂)₄-SH

unter 5 Mol.% ( 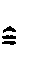 < 10 Gew.%).

### Beispiel 2

Entsprechend Beispiel 1 löst man 23,4 g (0,03 mol) Polytetrahydrofuran-bis-methansulfonat (s. Formel II mit n = 6,7 und R = CH₃) und 14,9 g Natriumthiosulfatpentahydrat (0,06 mol) in 100 ml Ethanol und 30 ml Wasser und erhitzt das Gemisch 6 h unter Rückfluß zum Sieden. Man kühlt auf Raumtemperatur ab und gibt 5 ml konz. Salzsäure in 35 ml Ethanol zu. Dann erhitzt man 1 weitere Stunde unter Rückfluß. Nach dem Abkühlen werden 100 ml Wasser zugegeben. Man schüttelt mit MTB aus. Nach dem Trocknen und Einengen der organischen Phase erhält man 19,6 g (99,6 %) Polybutandiol(1,4)ω,ω′bismercaptan in einer Reinheit von ca. 54%.
Analysen: Molgewicht (osm.): 900
¹H-NMR (CDCH₃): δ = 3,4 (m, Polyether-CH₂), 2,7 (m, α-CH₂ d. Thioethers), 2,56 (m,α, CH₂ d. Thiols), 1,6 (m, Polyether-CH₂), 1,35 (t, SH)

+Die Daten sprechen für einen Anteil an dem in Beispiel 1 genannten Thioether von ca. 30 Mol%( 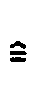 54 Gew.%).

## Patentansprüche

1. Polybutandiol(1,4)ω,ω′bismercaptane der allgemeinen Formel
HS-(CH₂)₄-O-[(CH₂)₄-O]ₙ-(CH₂)₄-SH I,
in der n eine Zahl von 1 bis 150 bedeutet.

2. Verfahren zur Herstellung von Polybutandiol(1,4)ω,ω′bismercaptanen nach Anspruch 1, dadurch gekennzeichnet, daß man Polytetrahydrofuranderivate der allgemeinen Formel 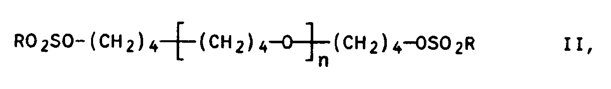 in der R einen Alkylrest mit 1 bis 4 C-Atomen oder einen Arylrest bedeutet und n die obengenannte Bedeutung hat, mit Schwefel abgebenden Verbindungen bei Temperaturen bis 150°C umsetzt.

3. Verfahren nach Anspruch 2, dadurch gekennzeichnet, daß man das Polytetrahydrofuranderivat der Formel II mit Thioharnstoff umsetzt.

## Claims

1. A poly(1,4-butanediol-ω,ω′-bismercaptan) of the general formula
HS-(CH₂)₄-O-[(CH₂)₄-O]ₙ-(CH₂)₄-SH I
where n is from 1 to 150.

2. A process for preparing a poly(1,4-butanediol-ω,ω′-bismercaptan) as claimed in claim 1, which comprises reacting a polytetrahydrofuran derivative of the general formula 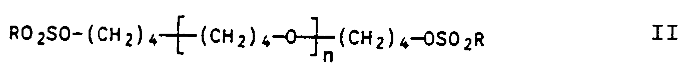 where R is alkyl of from 1 to 4 carbon atoms or aryl, and n is as defined above, with a sulfur-donating compound at up to 150°C.

3. A process as claimed in claim 1, wherein the polytetrahydrofuran derivative of the formula II is reacted with thiourea.

## Revendications

1. Polybutanediol(1,4)oméga,oméga′bismercaptans de la formule générale:
HS-(CH₂)₄-O-[(CH₂)₄-O]ₙ-(CH₂)₄-SH I,
dans laquelle n est un nombre de 1 à 150.

2. Procédé de préparation de polybutanediol(1,4)oméga, omega′bismercaptans selon la revendication 1, caractérisé en ce que l'on fait réagir des dérivés de polytétrahydrofurannes de la formule générale: 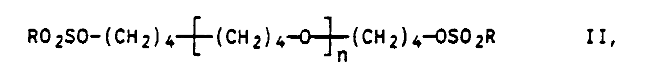 dans laquelle R représente un radical alkyle comportant de 1 à 4 atomes de carbone ou un radical aryle et n possède les significations qui lui ont été attribuées ci-dessus, avec des composés qui libèrent du soufre, à des températures allant jusqu'à 150°C.

3. Procédé selon la revendication 2, caractérisé en ce que l'on fait réagir le dérivé de polytétrahydrofuranne de la formule II avec la thiourée.
